# EUROPEAN PATENT SPECIFICATION

(11) **EP 3 047 816 B1**
(45) Date of publication and mention of the grant of the patent: **03.10.2018**
(21) Application number: 16151391.6
(22) Date of filing: 14.01.2016
(51) Int. Cl.: A61B 90/90

(54) **WIRELESSLY DETECTABLE OBJECTS FOR USE IN MEDICAL PROCEDURES**
DRAHTLOS ERKENNBARE OBJEKTE ZUR VERWENDUNG IN MEDIZINISCHEN EINGRIFFEN
OBJETS DETECTABLES SANS FIL A UTILISER DANS DES PROCEDURES MEDICALES

(30) Priority: 21.01.2015 US 201562106052 P; 25.03.2015 US 201562138248 P
(43) Date of publication of application: 27.07.2016
(73) Proprietor: Covidien LP, Mansfield, MA 02048 (US)
(72) Inventor: BLAIR, William A., Carlsbad, CA 92008 (US)
(74) Representative: CBDL Patentanwälte

(56) References cited:
- WO-A1-2004/054801
- US-A1- 2005 049 564
- US-A1- 2005 203 470
- US-A1- 2009 315 681
- US-A1- 2010 108 079

## Description

### BACKGROUND

### Technical Field

The present disclosure generally relates to detection of presence, or absence, and identification of objects tagged with wirelessly detectable objects, which may, for example, allow detection and/or identification of surgical objects (e.g., sponges, instruments, etc.) during or after surgery, or for inventorying of objects, for instance surgical objects.

### Description of the Related Art

It is often useful or important to be able to determine the presence or absence of an object.

For example, it is important to determine whether objects associated with surgery are present in a patient's body before completion of the surgery. Such objects may take a variety of forms. For example, the objects may take the form of instruments, for instance scalpels, scissors, forceps, hemostats, and/or clamps. Also for example, the objects may take the form of related accessories and/or disposable objects, for instance surgical sponges, gauzes, and/or pads. Failure to locate an object before closing the patient may require additional surgery, and in some instances may have serious adverse medical consequences.

Some hospitals have instituted procedures which include checklists or require multiple counts to be performed to track the use and return of objects during surgery. Such manual approaches are inefficient, requiring the time of highly trained personnel, and are prone to error.

Another approach employs transponders and a wireless interrogation and detection system. Such an approach employs wireless transponders which are attached to various objects used during surgery. The interrogation and detection system includes a transmitter that emits pulsed wideband wireless signals (e.g., radio or microwave frequency) and a detector for detecting wireless signals returned by the transponders in response to the emitted pulsed wideband signals. Such an automated system may advantageously increase accuracy while reducing the amount of time required of highly trained and highly compensated personnel. Examples of such an approach are discussed in U.S. Patent No. 6,026,818, issued February 22, 2000, and U.S. Patent Publication No. US 2004/0250819, published December 16, 2004.

However, some of these approaches do not allow identification of the object. Conventional approaches that allow identification of the object via transmitting an identifier typically transmit a signal at frequencies that have a short range of detection, which may inhibit detection of the transponder, and thus, the object attached thereto. Furthermore, these transponders may not be detectable by the interrogation device when they are situated such that there is an obstacle or membrane, such skin or flesh, between the transponder and the interrogation device.

Another example of a wirelessly detectable object is described in U.S. Patent Publication No. US 2005/0049564 A1, the content of which the preamble of claim 1 is based upon. Consequently, a new approach to uniquely identify and detect presence and absence of a transponder assembly as well as identification is desirable.

### BRIEF SUMMARY

The scope of the invention is defined by claim 1 and preferred embodiments are defined in the dependent claims. It may be useful for a medical provider to be able to detect a transponder at longer ranges while still being able to receive an identifier from the transponder to uniquely identify the object. For example, upon detecting that an object is present in a proximity of the surgical site, particularly inside the body of the patient, it may be useful to wirelessly determine an identity of the object. Further, upon completion of surgery, it may useful to scan the objects that were used during surgery and are currently present, to identify them and determine whether all of the objects that were present before surgery are present after surgery outside the patient's body without requiring a manual count of the objects by highly trained and highly compensated personnel.

Additionally, identification of the object can also be useful in counting a number of packaged objects at completion of a manufacturing process to ensure that an appropriate number of objects are included in a shipping tote or other package. Identification of the object may also be useful in determining use history of an object, or the duration of time lapsed from a reference point in time relating to the object, such as a last maintenance time of the object. For example, in the medical or surgical context, tools such as those listed above, can have a limited shelf life after being disinfected and before being used or reused. Furthermore, some tools have a total life cycle after which they need to be replaced or go through maintenance before being reused. Conventional manual tracking of an object's life cycle, maintenance cycle, shelf life or any other parameter, even when assisted by computers, can be costly and time-consuming.

Furthermore, certain surgical objects may undergo one or more rounds of sterilization before and/or after use within the surgical environment. For example, such sterilization procedures may include subjecting the surgical object to one or more of elevated temperatures and/or pressures, steam, irradiation, sterilizing chemicals, or other potentially hazardous or damaging environments.

If the transponder assembly is employed, for example, to track use and/or sterilization of the surgical object, the apparatus or structure that holds and/or physically couples the transponder to the object may remain attached to the surgical object during such sterilization procedures.

However, if the apparatus is unable to withstand such sterilization processes, the apparatus may become detached from the surgical object. Such detachment will interfere with the ability to use the transponder to track the surgical object. As another example, if the apparatus is unable to withstand such sterilization processes, the apparatus may insufficiently protect the transponder from certain hazards of the sterilization process.

Therefore, wireless transponder assemblies that are capable of withstanding different sterilization processes are desirable.

A wirelessly detectable object to use in medical procedures may be summarized as including at least a first transponder that wirelessly receives a first interrogation signal and wirelessly returns a first response signal; a surgical object; and a pouch comprising at least a first flexible layer that forms an interior cavity and an adhesive layer physically coupled to at least the first flexible layer about at least a portion of a perimeter of the interior cavity, the adhesive layer which retains structural and adhesive integrity at least at temperatures equal to 121 degrees Centigrade, the pouch physically coupled to at least a portion of the surgical object. The adhesive layer may retain structural and adhesive integrity at least at temperatures equal to 130 degrees Centigrade. The adhesive layer may retain structural and adhesive integrity at least at temperatures equal to 136 degrees Centigrade. The adhesive layer may retain structural and adhesive integrity at least at temperatures equal to 150 degrees Centigrade. The surgical object may include a piece of absorbent material. The adhesive layer may physically couple the pouch to at least the portion of the surgical object. The interior cavity may be formed between the first flexible layer and the adhesive layer.

The wirelessly detectable object may further include a second flexible layer physically coupled to the first flexible layer, the interior cavity formed between the first flexible layer and the second flexible layer. The second flexible layer may be physically coupled to the adhesive layer. The adhesive layer may be physically coupled to at least a portion of a first surface of the first flexible layer and the second flexible layer may be physically coupled to at least a portion of a second surface of the first flexible layer that is opposite the first surface. The adhesive layer may be physically coupled to at least a first surface of the first flexible layer about the perimeter of the interior cavity and the second flexible layer may be physically coupled to at least a second surface of the first flexible layer about the perimeter of the interior cavity, the second surface of the first flexible layer opposite the first surface of the first flexible layer. The adhesive layer may be continuously physically coupled to at least a first surface of the first flexible layer and the second flexible layer may be physically coupled to at least a second surface of the first flexible layer about the perimeter of the interior cavity, the second surface of the first flexible layer opposite the first surface of the first flexible layer. The adhesive layer may include a hot melt adhesive layer. The hot melt adhesive layer may include a high temperature hot melt adhesive layer. The hot melt adhesive layer may have a melting point temperature greater than a sterilization temperature associated with one or more sterilization procedures. The hot melt adhesive layer may have a melting point temperature greater than a steam temperature at which a volume of steam is maintained during one or more sterilization procedures. The hot melt adhesive layer may have a melting point temperature of greater than 136 degrees Centigrade. The hot melt adhesive layer may have a melting point temperature of about 150 degrees Centigrade or higher. The adhesive layer may include a meltable plastic layer. The adhesive layer may include a thermoplastic layer. The adhesive layer may be biocompatible. The adhesive layer may include an adhesive web film. The adhesive layer may include a thermal lamination film. The adhesive layer may include a thermosetting plastic layer that has an initial cure temperature at which the thermosetting plastic layer cures, the thermosetting plastic layer which retains structural and adhesive integrity at least at temperatures equal to 121 degrees Centigrade subsequent to curing. The adhesive layer may include a heat-activated adhesive layer. The adhesive layer may include a pressure-activated adhesive layer. The adhesive layer may include a water-activated adhesive layer. The adhesive layer may include at least one of thermoplastic polyurethane, silicone, polyamide, polyethersulfone, polyethylene, polypropylene, and ethylene vinyl acetate. The first transponder may be received and freely movable in the interior cavity. The first transponder may include a presence transponder that wirelessly receives the first interrogation signal and wirelessly returns the first response signal that does not contain identification information. The first transponder may include a radio frequency identification (RFID) transponder that wirelessly receives the first interrogation signal and wirelessly returns the first response signal that contains identification information associated with the wirelessly detectable object.

The wirelessly detectable object may further include a radio frequency identification (RFID) transponder that wirelessly receives a second interrogation signal and wirelessly returns a second response signal that contains identification information associated with the wirelessly detectable object, the presence transponder not directly physically attached to the RFID transponder. The RFID transponder may be received within the interior cavity. The RFID transponder may be received and freely movable within the interior cavity. The RFID transponder may form at least a portion of the first flexible layer, may be embedded within the first flexible layer, or may be adhered to the first flexible layer by the adhesive layer.

The pouch may further include a radio frequency (RF) weld that extends around a perimeter of the interior cavity, physically couples the first flexible layer to the adhesive layer, and seals the presence transponder within the interior cavity. The pouch may further include a radio frequency (RF) weld that extends around a perimeter of the interior cavity, physically couples the first flexible layer to the second flexible layer, and seals the presence transponder within the interior cavity. One or both of the first flexible layer and second flexible layer may be a fabric laminate. The fabric laminate may include thermoplastic polyurethane and nylon fabric or polyvinyl chloride (PVC) impregnated fabric.

A wirelessly detectable object to use in medical procedures may be summarized as including a radio frequency identification (RFID) transponder that wirelessly receives a first interrogation signal and wirelessly returns a first response signal that contains identification information associated with the wirelessly detectable object; a presence transponder that wirelessly receives a second interrogation signal and wirelessly returns a second response signal that does not contain identification information; a piece of absorbent material; and a pouch comprising at least a first flexible layer that forms an interior cavity, the presence transponder received and freely movable within the interior cavity, the presence transponder independently movable with respect to the RFID transponder, the pouch physically coupled to at least a portion of the piece of absorbent material. The presence transponder may not be directly physically attached to the RFID transponder. The RFID transponder may be received within the interior cavity. The RFID transponder may be received and freely movable within the interior cavity. The RFID transponder may form at least a portion of the first flexible layer, may be embedded within the first flexible layer, or may be adhered to the first flexible layer. The RFID transponder may include an RFID chip and an antenna trace. Either or both of the RFID chip and the antenna trace may be embedded within the first flexible layer.

The antenna trace of the RFID transponder may include an active antenna element, the wirelessly detectable object may further include a passive antenna element, and the active antenna element and the passive antenna element together may form a directional antenna. The passive antenna element may be embedded in the first flexible layer. The first flexible layer may be physically coupled to the piece of absorbent material to form the interior cavity therebetween and at least the active antenna element of the RFID transponder may be received within the interior cavity and adhered to the piece of absorbent material.

The pouch may further include a second flexible layer physically coupled to the first flexible layer to form the interior cavity therebetween, the second flexible layer different than the piece of absorbent material. The RFID transponder may form at least a portion of the second flexible layer, is embedded within the second flexible layer, or may be adhered to the second flexible layer.

The wirelessly detectable object may further include a passive antenna element embedded in or adhered to the first flexible layer, the passive antenna element and the RFID transponder together forming a directional antenna.

The pouch may further include a radio frequency (RF) weld that extends around a perimeter of the interior cavity, physically couples the first flexible layer to the second flexible layer, and seals the presence transponder within the interior cavity.

The RF weld may include a first RF weld and wherein the first RF weld or a second RF weld may further physically couple the pouch to the piece of absorbent material. One or both of the first flexible layer and second flexible layer may be a fabric laminate.

The pouch may further include a radio frequency (RF) weld that extends around a perimeter of the interior cavity, physically couples the first flexible layer to the piece of absorbent material, and seals the presence transponder within the interior cavity. The first flexible layer may be formed of a fabric laminate. The fabric laminate may include thermoplastic polyurethane and nylon fabric or polyvinyl chloride (PVC) impregnated fabric.

A wirelessly detectable object to use in medical procedures may be summarized as including a piece of absorbent material; a first substrate physically coupled to the piece of absorbent material; a radio frequency identification (RFID) transponder to wirelessly receive a first interrogation signal and wirelessly return a first response signal that contains identification information associated with the wirelessly detectable object, the RFID transponder comprising an active antenna element; and a passive antenna element; wherein the passive antenna element and the active antenna element together operate as a directional antenna and the first substrate carries at least one of the active antenna element and the passive antenna element. The first substrate may include a layer of fabric laminate.

The fabric laminate may be physically coupled to the piece of absorbent material to form an interior cavity therebetween and the wirelessly detectable object may further include a presence transponder received and freely movable within the interior cavity, the presence transponder to wirelessly return a second response signal that does not contain identification information. The RFID transponder may be embedded in or adhered to the layer of fabric laminate or may be received within the interior cavity and adhered to the piece of absorbent material. The passive antenna element may be located between the piece of absorbent material and the layer of fabric laminate and the active antenna element may be embedded in, adhered to, or forms a portion of the layer of fabric laminate. The active antenna element may be located between the piece of absorbent material and the layer of fabric laminate and the passive antenna element may be embedded in, adhered to, or forms a portion of the layer of fabric laminate. The layer of fabric laminate may be carried at least in part by one or more of the passive antenna element and the active antenna element.

The wirelessly detectable object may further include a second layer of fabric laminate located between the passive antenna element and the piece of absorbent material.

The wirelessly detectable object may further include a presence transponder physically coupled to the piece of absorbent material, the presence transponder to wirelessly return a second response signal that does not contain identification information. The directional antenna may include a Yagi antenna. One or both of the active antenna element and the passive antenna element may include conductive traces embedded within or carried on the first substrate.

An exemplary method not forming part of the invention, to account for surgical objects used in medical procedures may be summarized as including providing a plurality of surgical objects that have a plurality of wirelessly detectable objects respectively physically coupled thereto, each wirelessly detectable object comprising a radio frequency identification (RFID) transponder and a presence transponder; interrogating the RFID transponder of each surgical object introduced into a surgical field; receiving, from the interrogated RFID transponder of each surgical object introduced into the surgical field, a first response signal that contains identification information stored by such RFID transponder; generating a first manifest of surgical objects introduced into the surgical field based at least in part on the identification information included in each first response signal; prior to completion of a medical procedure, scanning the surgical field to interrogate any presence transponders that remain within the surgical field; determining whether any surgical objects remain within the surgical field based at least in part on whether one or more second response signals are respectively received from one or more presence transponders responsive to the scanning, wherein the one or more second response signals do not contain identification information; interrogating the RFID transponder of each surgical object removed from the surgical field; receiving, from the interrogated RFID transponder of each surgical object removed from the surgical field, a third response signal that contains the identification information stored by such RFID transponder; and generating a second manifest of surgical objects removed from the surgical field based at least in part on the identification information included in each third response signal. Receiving a first response signal may include receiving the first response signal that is within a first frequency range, and determining whether any surgical objects remain within the surgical field may include determining whether any surgical objects remain within the surgical field based at least in part on whether one or more second response signals are respectively received from one or more presence transponders responsive to the scanning, the one or more second response signals within a second frequency range that provides superior transmission through bodily tissue relative to the first frequency range. Receiving a first response signal may include receiving the first response signal at a first physical distance from each RFID transponder, and determining whether any surgical objects remain within the surgical field may include determining whether any surgical objects remain within the surgical field based at least in part on whether one or more second response signals are respectively received at a second physical distance from one or more presence transponders responsive to the scanning, the second physical distance greater than the first physical distance.

The exemplary method may further include comparing the first manifest to the second manifest to determine whether one or more surgical objects remain within the surgical field.

### BRIEF DESCRIPTION OF THE SEVERAL VIEWS OF THE DRAWINGS

In the drawings, identical reference numbers identify similar elements or acts. The sizes and relative positions of elements in the drawings are not necessarily drawn to scale. For example, the shapes of various elements and angles are not necessarily drawn to scale, and some of these elements may be arbitrarily enlarged and positioned to improve drawing legibility. Further, the particular shapes of the elements as drawn, are not necessarily intended to convey any information regarding the actual shape of the particular elements, and may have been solely selected for ease of recognition in the drawings.
Figure 1A is a schematic diagram illustrating a surgical environment where a medical provider uses an interrogation and detection system to detect an object tagged with a wirelessly detectable object in a patient, according to one illustrated embodiment.
Figure 1B is an isometric view of a surgical object tagged with a wirelessly detectable object, according to one illustrated embodiment.
Figure 2A is a front view of a pouch that includes a presence transponder, according to one illustrated embodiment.
Figure 2B is a front view of another pouch that includes a presence transponder, according to one illustrated embodiment.
Figure 3 is a front view of a piece of absorbent material with a wirelessly detectable object physically coupled thereto, according to one illustrated embodiment.
Figure 4 is a front view of a pouch that includes a presence transponder freely movable within an interior cavity and an RFID transponder, according to one illustrated embodiment.
Figure 5A is a top view of a pouch, according to one illustrated embodiment.
Figure 5B is an exploded isometric view of a pouch that includes a presence transponder freely movable within an interior cavity and an RFID transponder adhered to a second layer of the pouch, according to one illustrated embodiment.
Figure 5C is first and second exploded side views of a pouch that includes a presence transponder freely movable within an interior cavity and an RFID transponder adhered to a second layer of the pouch, according to one illustrated embodiment.
Figure 6A is a top view of a pouch, according to one illustrated embodiment.
Figure 6B is an exploded isometric view of a pouch that includes a presence transponder and an RFID transponder freely movable within an interior cavity, according to one illustrated embodiment.
Figure 6C is first and second exploded side views of a pouch that includes a presence transponder and an RFID transponder freely movable within an interior cavity, according to one illustrated embodiment.
Figure 7 is a cross-sectional diagram of a wirelessly detectable object that includes a directional antenna formed on or within a pouch, according to one illustrated embodiment.
Figure 8 is a cross-sectional diagram of a wirelessly detectable object that includes a directional antenna carried at least in part by a first substrate, according to one illustrated embodiment.
Figure 9 is a cross-sectional diagram of a wirelessly detectable object that includes a directional antenna carried at least in part by each of a first and second substrate, according to one illustrated embodiment.
Figure 10 is a schematic diagram of an exemplary method for manufacturing wirelessly detectable objects using RF welding.
Figure 11 shows flexible layers usable to manufacture a plurality of pouches, according to one illustrated embodiment.
Figure 12 shows manufacture of a plurality of pouches using an RF welding technique, according to one illustrated embodiment.
Figure 13 is a front view of a plurality of pouches manufactured using an RF welding technique, according to one illustrated embodiment.

### DETAILED DESCRIPTION

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures associated with transmitters, receivers, or transceivers, and types of objects employed in medical procedures, for instance sponges, gauze or other absorbent objects, have not been shown or described in detail to avoid unnecessarily obscuring descriptions of the embodiments.

Unless the context requires otherwise, throughout the specification and claims which follow, the word "comprise" and variations thereof, such as "comprises" and "comprising," are to be construed in an open, inclusive sense, as "including, but not limited to."

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The headings and Abstract of the Disclosure provided herein are for convenience only and do not interpret the scope or meaning of the embodiments.

For ease of understanding, a surgical environment will be used as an example environment for detecting objects but such should not be considered limiting. Finally, the scope of the subject-matter of the invention is defined only by the appended claims. Additional disclosures and descriptions not belonging to this scope are provided for better understanding of the invention and its context.

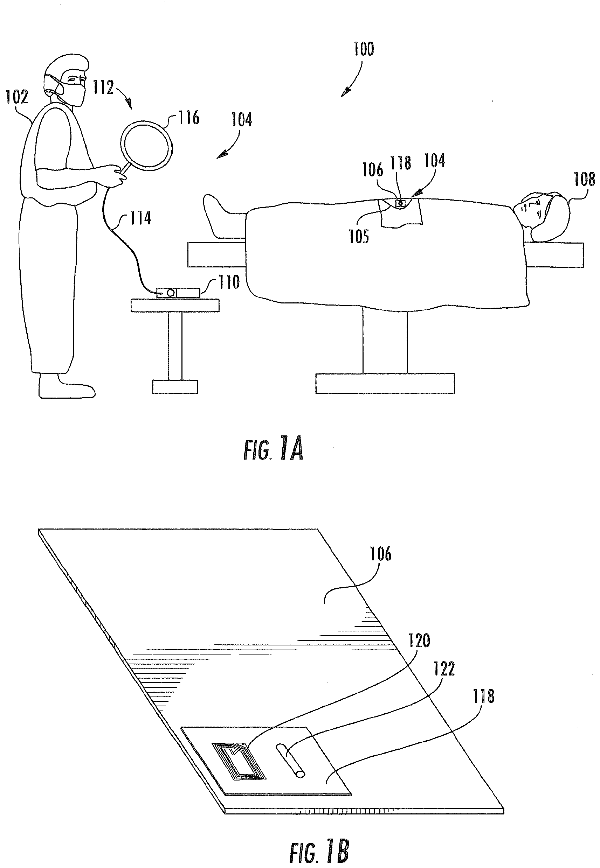

Figure 1A shows a surgical environment 100 in which medical procedures are performed, for example a surgical environment, clinician's office, examination room, patient room or other environments in which medical procedures may be performed. A medical provider 102 operates an identification and detection system 104 to ascertain the presence or absence of objects 106 in, or on, a patient 108, for example in or on a surgical site or area or cavity 105, and/or an identity of such objects 106.

The object 106 may take a variety of forms, for example instruments, accessories and/or disposable objects useful in performing surgical procedures. For instance, the object 106 may take the form of scalpels, scissors, forceps, hemostats, dilators, needles, a drill bit, and/or clamps or other surgically useful objects. Also for example, the objects 106 may take the form of surgical sponges, gauze and/or padding. The surgical sponges, gauze and/or padding may be, as examples, 5.08 cm by 5.08 cm, 10.16 cm by 10.16 cm, 30.48 cm by 30.48 cm (2 inches by 2 inches, 4 inches by 4 inches, 12 inches by 12 inches), or other sizes. Such dimensions may refer to the surgical sponges, gauze and/or padding as folded or otherwise packaged.

According to an aspect of the present disclosure, the object 106 is tagged, carrying, attached or otherwise coupled to a wirelessly detectable object 118.

In particular, referring now to Figure 1B, a wirelessly detectable object 118 is physically coupled to or otherwise physically associated with each object 106 used within the surgical environment 100. The wirelessly detectable object 118 includes one or more transponders that receive and respond to wireless signals. For example, in some implementations, the wirelessly detectable object 118 includes a radio frequency identification (RFID) transponder 120 that, when interrogated, wirelessly returns a first response signal that contains identification information associated with the wirelessly detectable object 118. Alternatively or additionally, the wirelessly detectable object 118 includes a presence transponder 122 that, when interrogated, wirelessly returns a second response signal that does not contain identification information.

Thus, in some implementations, the medical provider 102 can operate the identification and detection system 104 to determine the presence or absence of wirelessly detectable object 118 through wireless interrogation of the presence transponder 122 and/or to obtain identification information through wireless interrogation of the RFID transponder 120. In particular, in some implementations, respective interrogation of and response by the presence transponder 122 and the RFID transponder 120 can occur in two different frequency ranges. For example, the frequency range associated with interrogation of and response by the presence transponder 122 can include lower frequencies than the frequency range associated with interrogation of and response by the RFID transponder 120. Such lower frequencies may enable superior transmission of signals through bodily tissues or other obstacles including membranes, skin, flesh, etc. Thus, in some implementations, interrogation of and response by the presence transponder 122 is possible at larger physical distances than interrogation of and response by the RFID transponder 120.

The RFID transponder 120 includes an integrated circuit electrically coupled to an antenna. The RFID transponder 120 may be relatively small, such as, for example, approximately 12 millimeters in diagonal.

In some implementations, the antenna can include an inductive winding such as a conductive wire wound about a core. The core can be fabricated from a ferrite rod. The inductive winding is electrically coupled to an integrated circuit. In other implementations, the antenna includes a conductive trace or other structures. The RFID transponder 120 may be an active device that includes a local power source such as a battery or may be a passive device that relies on energy in the interrogation signal to power the transponder 120. In one aspect, the RFID transponder 120 takes the form of any one of various commercially-available RFID devices that include an RFID integrated circuit and/or front end.

The RFID transponder 120 is operable to transmit (e.g., via active radiation of the antenna) a first response signal that contains identification information, in response to receiving an interrogation signal in a first frequency range. The first response signal encodes the identification information stored by the integrated circuit. As such, the RFID transponder 122 may be denominated as a "smart" transponder.

The identification information included in the first response signal may be a unique identifier (i.e., unique over a set of all otherwise identical RFID transponders 120). Alternatively, the identifier may not be unique, for example, a set of RFID transponders 120 may each have the same identifier. Even where the identifier is unique, some portion of the identification information or some other identification information may not be unique, for example, a portion representing a manufacturer, a lot, or a type, may be shared between transponders 120 from the same manufacturer, lot or of the same type. In some implementations, the identification information can be associated with a type of the object 106 or an attribute thereof. For example, the identification information can be linked to the type or attribute using a database, lookup table, or other data structure that cross-references unique identifiers with the type or attribute.

Alternatively, in implementations where the integrated circuit of the RFID transponder 120 has read and write capability, the identification information can include the desired attribute, pre-stored or written onto the integrated circuit, and directly convey the pre-stored attribute via the first response signal.

Furthermore, in some implementations, the RFID transponder 120 is a printable and/or ultra-low-cost RFID transponder 120 that is not necessarily intended to maintain functionality when the object 106 is used within the surgical environment 100. In particular, in such implementations, the RFID transponder 120 is interrogated at a conclusion of or during a manufacturing process, for example, to ensure that an appropriate number of objects 106 are included in a shipping tote or other package. After such use, the RFID transponder 120 may not be expected to provide further use and may allowably degrade or otherwise experience damage if the object 106 is used within the surgical environment 100 (e.g., in vivo). Such may permit inclusion of low-cost RFID transponders 120 for use in manufacturing without requiring a hardened or rugged encapsulant or transponder body to protect the transponders 120 during surgical procedures.

The presence transponder 122 may be constructed in various manners. For example, the presence transponder 122 may include a ferrite rod with a conductive coil wrapped about an exterior surface thereof to form an inductor, and a capacitor coupled to the conductive coil to form a series circuit. The conductive coil may, for example, take the form of a spiral wound conductive wire with an electrically insulative sheath or sleeve. For example, the inductive coil and capacitor may together form an inductive/capacitance (L/C) tank circuit. Additional details about types of transponders may be found in U.S. Provisional Patent Application Serial No. 60/811,376 filed June 6, 2006 and U.S. Provisional Patent Application Serial No. 60/892,208, filed February 28, 2007.

The presence transponder 122 is operable to transmit (e.g., via radiation of the inductive coil) a second response signal, in response to receiving an interrogation signal in a second frequency range. The second response signal does not include any unique identifying information and, therefore, indicates only that the presence transponder 122 is present. As such, the presence transponder 122 may be denominated as a "dumb" transponder. However, in some implementations, presence transponder 122 provides superior response strength through bodily tissue relative to the RFID transponder 120.

The presence transponder 122 may be relatively small, for example approximately 5-10 millimeters long with a diameter of about 1-4 millimeters. In at least some embodiments, an encapsulant advantageously protects the transponder from the ambient environment, for instance from forces, pressure and/or fluids, such as bodily fluids.

In some implementations, the presence transponder 122 includes a dumbbell-shaped ferrite rod having broad end portions and a narrow intermediate portion. The broad end portions may provide capacitive functionality. In other implementations, the presence transponder 122 may be shaped as a fusiform-shaped object, with truncated ends.

In further implementations, the wirelessly detectable object 118 includes at least one directional antenna. For example, in some implementations, an active antenna element of the RFID transponder 120 forms at least a portion of the directional antenna. In some implementations, the wirelessly detectable object does not include the presence transponder 122. Particular example structures and arrangements of the wirelessly detectable object 118 are discussed further below with reference to the Figures that follow.

Furthermore, although Figure 1B depicts the wirelessly detectable object 118 as physically coupled to and visible upon an external surface of the object 106, such depiction is provided for ease of illustration and description only. In particular, in instances in which the object 106 is a piece of absorbent material such as surgical sponges, gauze, padding, or other absorbent materials, the piece of absorbent material 106 may be folded or otherwise manipulated such that the wirelessly detectable object 118 is no longer carried on an external surface of the piece of absorbent material 106 and/or externally visible. As an example, the piece of absorbent material 106 may be folded into quadrants to provide, for example, a folded sponge, gauze, or padding that has four discernable layers. As a result of the folding, the wirelessly detectable object 118 may be internally carried between layers of the piece of absorbent material 106 and visible only upon unfolding of the piece of absorbent material 106.

In addition, according to an aspect of the present disclosure, the wirelessly detectable object 118 may include an adhesive layer that physically couples the wirelessly detectable object 118 to the piece of absorbent material 106 or other surgical object 106. The adhesive layer may retain structural and adhesive integrity at least at temperatures equal to 121 degrees Centigrade, 130 degrees Centigrade, 132 degrees Centigrade, 136 degrees Centigrade, and/or 150 degrees Centigrade, or higher.

For example, the adhesive layer may not melt or otherwise liquefy and may retain adhesion to the remainder of the wirelessly detectable object 118 and/or the surgical object 106 at temperatures less than or equal to 121, 130, 132, 136, and/or 150 degrees Centigrade or higher. In some implementations, the adhesive layer may retain the structural and adhesive integrity at least at temperatures equal to 150 degrees Centigrade or higher.

As an example, the adhesive layer may be a hot melt adhesive layer positioned between the surgical object 106 and the remainder of the wirelessly detectable object 118. In such implementations, the wirelessly detectable object 118 may be physically coupled to the surgical object 106 by causing the temperature of at least a portion the hot melt adhesive layer to exceed a melting point temperature associated with the hot melt adhesive layer, thereby causing such portion to at least in part melt. For example, such may be performed using an RF welding machine, planar heat pressing machine, hot-air welding machine, or laminator. Alternatively, the wirelessly detectable object 118 and the surgical object 106 may be baked (e.g., in a chamber) or exposed to various other techniques for applying heat and/or pressure at desired locations. Generally, the melting point temperature will be at least greater than 121 degrees Centigrade, but may be other temperatures in various implementations.

Thus, for example, in contrast to an epoxy that is applied in liquid form and then cured, the adhesive layer of the wirelessly detectable object 118 may be a pre-formed solid layer that is positioned or laid between the remainder of the wirelessly detectable object 118 and the surgical object 106. The adhesive layer may then be caused to at least in part melt and then re-solidify, thereby engaging the remainder of the wirelessly detectable object 118 and the surgical object 106 and resulting in physical coupling therewith.

In some implementations, the hot melt adhesive layer is a high temperature hot melt adhesive layer (i.e., a hot melt adhesive layer that has a relatively high melting point temperature). For example, the hot melt adhesive layer may have a melting point temperature of greater than 121 degrees Centigrade, greater than 130 degrees Centigrade, greater than 132 degrees Centigrade, or greater than 136 degrees Centigrade. As another example, the hot melt adhesive layer may have a melting point temperature of about 150 degrees Centigrade or higher.

More particularly, according to an aspect of the present disclosure, the hot melt adhesive layer may have a melting point temperature greater than a sterilization temperature associated with one or more sterilization procedures. For example, the hot melt adhesive layer may have a melting point temperature greater than a steam temperature at which a volume of steam is maintained during one or more steam-based sterilization procedures. For example, two common steam-based sterilization techniques use a volume of steam respectively maintained at 121 degrees Centigrade (250 degrees Fahrenheit) and 132 degrees Centigrade (270 degrees Fahrenheit). The hot melt adhesive layer may have a melting point temperature greater than one or both of such temperatures.

Further, certain sterilization procedures may be performed with pressure conditions greater than 1 atmosphere. The hot melt adhesive layer may any of the melting point temperature characteristics described herein at such pressure conditions.

In some implementations, the adhesive layer is biocompatible, permitting use of the wirelessly detectable object 118 in vivo. In some implementations, the adhesive layer is an adhesive web film. In some implementations, the adhesive layer is a thermal lamination film. The adhesive layer may be a meltable plastic layer, such as, for example, a thermoplastic layer.

In some implementations, the adhesive layer may be a thermosetting plastic layer that has an initial cure temperature at which the thermosetting plastic layer cures. For example, the initial cure temperature may be less than 130 degrees Centigrade. Subsequent to curing, the thermosetting plastic layer may retain structural and adhesive integrity at least at temperatures less than or equal to 121, 130, 132, 136, and/or 150 degrees Centigrade or higher.

In some implementations, the adhesive layer may be a heat-activated adhesive layer. Alternatively or additionally, the adhesive layer may be a pressure-activated adhesive layer or a pressure-sensitive adhesive layer. Alternatively or additionally, the adhesive layer may be a water-activated adhesive layer.

The adhesive layer may include at least one of thermoplastic polyurethane, silicone, polyamide, polyethersulfone, polyethylene, polypropylene, and ethylene vinyl acetate.

Referring again to Figure 1A, the identification and detection system 104 includes a controller 110, and an interrogation device or assembly, such as an antenna 112 coupled to the controller 110 by one or more communication paths, for example a coaxial cable 114. The antenna 112 may take the form of a hand-held wand 116. In some implementations, the antenna 112 is sized to fit at least partially in the cavity 105.

The controller 110 is configured to cause the antenna 112 to emit one or more wireless interrogation signals in one or more frequency bands, to receive responses to such interrogation signals from one or more wirelessly detectable objects 118, and to determine the presence or absence and/or identity of the wirelessly detectable objects 118 or associated objects 106 based on the received response signals, if any.

In particular, the wand 116 can be configured to emit a first interrogation signal in a first frequency range and can include an integrated circuit tag reader, such as an RFID reader as is known, to receive the first response signal from the RFID transponder 120 and decode the identifier. The wand 116 can further be configured to emit a second interrogation signal in a second frequency, to receive the second response signal from the presence transponder 122, and to provide an indication of presence of the object 106 when the second response signal is received.

Specific details of components of the wand 116 are not discussed herein to not unnecessarily obscure the description of the embodiments. Components configured for emission of the interrogation signals and for receiving the first and second response signals can be selected from any suitable scanning technology, including, but not limited to, the detection device disclosed in U.S. Patent No. 6,026,818, to Blair et al., and that disclosed in U.S. Patent No. 7,696,877, to Barnes et al..

Furthermore, in some implementations, the controller 110 of the interrogation device or assembly includes an interface that displays the name of the objects 106 as the wand 116 scans the objects 106 after surgery. For example, the interface may display an accounting or inventory of sponges, gauzes, padding, hemostats, clamps, forceps, scissors, scalpels, or other surgical tools or accessories, or any other objects 106, for an expedient accounting of the objects 106.

As one example method of operation, a user, such as the medical provider 102, can scan the patient 108 to detect presence or absence of wirelessly detectable objects 118 and their corresponding objects 106 within the patient 108 through wireless interrogation of one or more presence transponders 122. For example, such interrogation of the presence transponders 122 can occur at a first physical distance. Upon detecting the presence of an object 106 within the patient 108, the medical provider 102 can immediately scan the region of detection to wirelessly interrogate one or more RFID transponders 120 and thereby identify the one or more objects 106 that remain. For example, such interrogation of the RFID transponders 120 can occur at a second physical distance that is less than the first physical distance. Having obtained the identity of the object 106, the medical provider 102 can make informed decisions with respect to handing of the object 106. For example, the medical provider 102 can remove object prior to closing patent.

As another example, upon removing the object or objects 106 from the body of the patient 108, and with all the present objects 106 laid out in an area after surgery and before closing the surgical site or area 105, the medical provider 102 can scan the present objects 106 to ensure that all the objects 106 that were present before surgery, are now present and outside of the body of the patient 108 after surgery. For example, the medical provider can interrogate the RFID transponder 120 of each wirelessly detectable object 118 to identify all present objects 106. The presently identified objects 106 can be compared to a list of objects 106 identified and logged prior to use within the surgical environment to detect any discrepancies (i.e., missing objects).

As yet another example method of operation, one or more RFID transponders 120 for one or more objects 106 may be interrogated at a conclusion of or during a manufacturing process, for example, to ensure that an appropriate number of objects 106 are included in a shipping tote or other package. Upon entry into and use of the objects 106 within the surgical environment, the RFID transponders 120 may or may not degrade. However, the medical provider 102 may still interrogate one or more presence transponders 122 to advantageously detect presence or absence of wirelessly detectable objects 118 and their corresponding objects 106 within the patient 108.

Accordingly, the wirelessly detectable objects 118 of the present disclosure provide the capability to efficiently detect objects 106 that may be present in or on the body of the patient 108, and the capability to conduct an inventory of present objects 106 after surgery to ensure all objects 106 used during surgery are present, without the use of multiple separately affixed optically-readable tags and without the need to conduct a manual count of the objects by highly trained and highly paid personnel.

Further, although a human patient 108 is illustrated, the described interrogation and detection system 104 may similarly be used on animals or inanimate subjects.

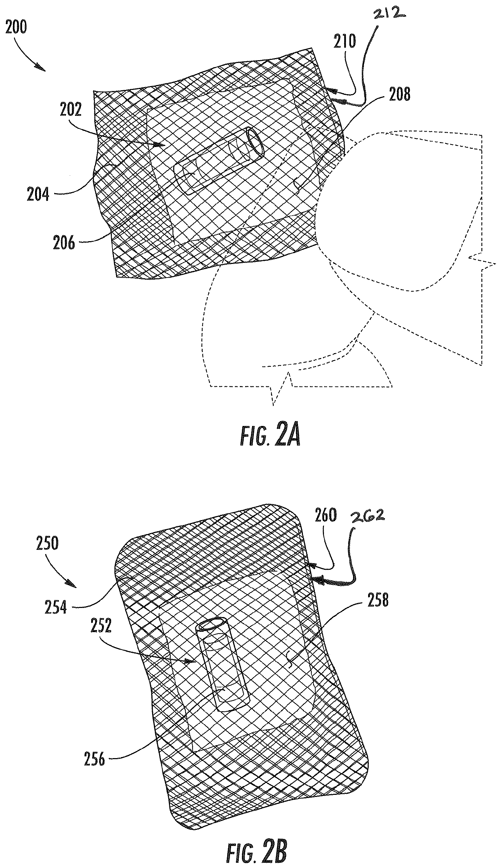

Figure 2A is a front view 200 of a pouch 202 that includes a presence transponder 206, according to one illustrated embodiment. In particular, in some implementations of the present disclosure, the wirelessly detectable object 118 includes a pouch 202 that holds or otherwise retains a presence transponder 206 within an interior cavity of the pouch 202. The pouch 202 is physically coupleable to an object 106 such as a piece of absorbent material.

In some implementations, the presence transponder 206 is freely movable within the interior cavity of the pouch 202. Such may advantageously allow folding, stretching, compression, twisting, or other physical manipulation of the piece of absorbent material or other object 106 without causing damage to the presence transponder 206. For example, the presence transponder 206 freely moves within the pouch 202 to an advantageous position experiencing reduced forces. Likewise, the free-floating presence transponder 206 does not inhibit folding, stretching, compression, twisting, or other physical manipulation of the piece of absorbent material or other object 106 which may be necessary for the surgical procedure.

The pouch 202 includes at least a first flexible layer 208 that forms the interior cavity. For example, the first flexible layer 208 can be physically coupled to a surface of an object 106 such as a piece of absorbent material to form the interior cavity therebetween. As another example, as shown in Figure 2A, the pouch 202 includes a second flexible layer 210 opposite the first flexible layer 208 and physically coupled to the first flexible layer 208 to form the interior cavity therebetween.

In the illustrated embodiment, the pouch 202 further includes an adhesive layer 212 positioned opposite the second flexible layer 210 from the first flexible layer 208. The adhesive layer 212 may be physically coupled to one or both of the first flexible layer 208 and the second flexible layer 210. Furthermore, in some implementations, the adhesive layer 212 physically couples the pouch 202 to a piece of absorbent material or other object 106.

According to an aspect of the present disclosure, the adhesive layer 212 may retain structural and adhesive integrity at least at temperatures equal to 121, 130, 132, 136, and/or 150 degrees Centigrade or higher. For example, the adhesive layer 212 may not melt or otherwise liquefy and may retain adhesion to the first flexible layer 208, second flexible layer 210 and/or the piece of the absorbent material 106 at temperatures less than or equal to 121, 130, 132, 136, and/or 150 degrees Centigrade or higher.

In some implementations, the adhesive layer 212 is physically coupled to at least a portion of a first surface of the second flexible layer 210 and the first flexible layer 208 is physically coupled to at least a portion of a second surface of the second flexible layer 210 that is opposite the first surface. In particular, in some implementations, the adhesive layer 212 is physically coupled to at least the first surface of the second flexible layer 210 about a perimeter of the interior cavity and the first flexible layer 208 is physically coupled to at least the second surface of the second flexible layer 210 about the perimeter of the interior cavity. In such implementations, the interior cavity may be formed between the first flexible layer 208 and the second flexible layer 210, as illustrated, or may be formed between the second flexible layer 210 and the adhesive layer 212.

In other implementations, the adhesive layer 212 is continuously physically coupled to at least the first surface of the second flexible layer 210 and the first flexible layer 208 is physically coupled to at least the second surface of the second flexible layer 210 about the perimeter of the interior cavity. In such implementations, the interior cavity may be formed between the first flexible layer 208 and the second flexible layer 210, as illustrated.

In yet other implementations, the pouch 202 includes the adhesive layer 212, but does not include the second flexible layer 210. In such implementations, the first flexible layer 208 is physically coupled to the adhesive layer 212. For example, the first flexible layer 208 may be physically coupled to the adhesive layer 212 at least about the perimeter of the interior cavity to form the interior cavity therebetween.

In some implementations, a radio frequency (RF) weld 204 physically couples the first flexible layer 208 to one or both of the second flexible layer 210 and the adhesive layer 212. For example, the RF weld 204 extends around a perimeter of the interior cavity and seals the presence transponder 206 within the pouch 202. A width of the RF weld 204 can be varied to balance various objectives such as a strength of weld 204 and a size of the pouch 202. Alternatively or additionally to RF weld 204, adhesives, stitching, clamping, fasteners, or other securing means can physically couple the first flexible layer 208 to the object 106 or the second flexible layer 210.

The first and/or second flexible layers 208 and 210 may be fabric laminates or other materials. For example, the first and/or second flexible layers 208 and 210 may be one or more of thermoplastic polyurethane (TPU) and nylon fabric; polyvinyl chloride (PVC) impregnated fabric; layer(s) of PVC, TPU, PET, PETG, LDPE, EVA, open celled polyurethanes, or nylon; other fabrics (e.g., cotton, polyester, leather, vinyl, polyethylene, and blended fabrics); other plastics; or combinations thereof. The flexible layers 208 and 210 are typically relatively thin and may be absorbent or non-absorbent. In some implementations, the flexible layers are of material suitable to prevent entry of fluids into the interior cavity of the pouch 202 (e.g., due to a water-proof or water-resistant coating). Thus, the first and/or second flexible layers 208 and 210 may be soft, pliable, and resistant to ripping or tearing.

In one particular example, the first flexible layer 208 includes a first layer of TPU and a first layer of nylon fabric. The second flexible layer 210 includes a second layer of TPU and a second layer of nylon fabric. For example, the first and second layers of TPU may respectively be located interior relative to the first and second layers of nylon fabric. In other words, the first and second layers of TPU may contact each other and may form an interior surface of the interior cavity of the pouch 202 while the first and second layers of nylon fabric are respectively carried by respective exterior surfaces of the first and second layers of TPU that are opposite to the interior cavity. Such may advantageously allow the first and second layers of TPU to more completely melt together or otherwise physically couple to each other when the RF weld 204 is generated. However, in other implementations, the first and second layers of nylon fabric may be located interior relative to the first and second layers of TPU or may be embedded within the first and second layers of TPU.

In some implementations, the adhesive layer 212 is a hot melt adhesive layer 212. In such implementations, the pouch 202 may be constructed at least in part by causing the temperature of at least a portion the hot melt adhesive layer 212 to exceed a melting point temperature associated with the hot melt adhesive layer 212, thereby causing such portion to at least in part melt. For example, such may be performed using an RF welding machine, planar heat pressing machine, hot-air welding machine, or laminator. Alternatively, the pouch 202 may be baked (e.g., in a chamber) or exposed to various other techniques for applying heat and/or pressure at desired locations. Generally, the melting point temperature will be at least greater than 130 degrees Centigrade.

Thus, for example, in contrast to an epoxy that is applied in liquid form and then cured, the adhesive layer 212 may be a pre-formed solid layer that is positioned or laid adjacent to the first and/or the second flexible layers 208 and 210 and then caused to at least in part melt and then re-solidify, thereby engaging the first and/or the second flexible layers 208 and 210 and resulting in physical coupling therewith. For example, in some implementations, the second layer 210 is a porous fabric and the adhesive layer 212 melts through the pores of the fabric to engage the first flexible layer 208. Such may result in physical coupling of the first flexible layer 208 to the second flexible layer 210 by way of the adhesive layer 212. Further, in some implementations, the adhesive layer 212 may be caused to at least in part melt, engage a piece of material or other object 106, and then re-solidify, resulting in physical coupling of the pouch 202 to the object 106.

In some implementations, the hot melt adhesive layer 212 is a high temperature hot melt adhesive layer 212 (i.e., a hot melt adhesive layer that has a relatively high melting point temperature). For example, the hot melt adhesive layer 212 may have a melting point temperature of greater than 121, 130, 132, or 136 degrees Centigrade. As another example, the hot melt adhesive layer 212 may have a melting point temperature of about 150 degrees Centigrade or higher.

More particularly, according to an aspect of the present disclosure, the hot melt adhesive layer 212 may have a melting point temperature greater than a sterilization temperature associated with one or more sterilization procedures. For example, the hot melt adhesive layer may have a melting point temperature greater than a steam temperature at which a volume of steam is maintained during one or more steam-based sterilization procedures. For example, two common steam-based sterilization techniques use a volume of steam respectively maintained at 121 degrees Centigrade (250 degrees Fahrenheit) and 132 degrees Centigrade (270 degrees Fahrenheit). The hot melt adhesive layer 212 may have a melting point temperature greater than one or both of such temperatures.

Further, certain sterilization procedures may be performed with pressure conditions greater than 1 atmosphere. The hot melt adhesive layer 212 may any of the melting point temperature characteristics described herein at such pressure conditions.

In some implementations, the adhesive layer 212 is biocompatible, permitting use of the wirelessly detectable object in vivo. In some implementations, the adhesive layer 212 is an adhesive web film. In some implementations, the adhesive layer 212 is a thermal lamination film. The adhesive layer 212 may be a meltable plastic layer, such as, for example, a thermoplastic layer.

In some implementations, the adhesive layer 212 may be a thermosetting plastic layer that has an initial cure temperature at which the thermosetting plastic layer cures. For example, the initial cure temperature may be less than 130 degrees Centigrade. Subsequent to curing, the thermosetting plastic layer may retain structural and adhesive integrity at least at temperatures less than or equal to 121, 130, 132, 136, and/or 150 degrees Centigrade or higher.

In some implementations, the adhesive layer 212 may be a heat-activated adhesive layer. Alternatively or additionally, the adhesive layer 212 may be a pressure-activated adhesive layer or a pressure-sensitive adhesive layer. Alternatively or additionally, the adhesive layer 212 may be a water-activated adhesive layer.

The adhesive layer 212 may include at least one of thermoplastic polyurethane, silicone, polyamide, polyethersulfone, polyethylene, polypropylene, and ethylene vinyl acetate.

In one particular example pouch 202, the first flexible layer 208 is a nylon layer; the second flexible layer 210 is a TPU layer; and the adhesive layer 212 is a hot melt adhesive layer. In some implementations, the pouch 202 does not include the adhesive layer 212.

Figure 2B is another front view 250 of a pouch 252 that includes a presence transponder 256, according to one illustrated embodiment. In particular, pouch 252 includes a first flexible layer 258 physically coupled to a second flexible layer 260 by an RF weld 254. The presence transponder 256 is received and freely movable within an interior cavity formed between the first and second flexible layers 258 and 260. In particular, the RF weld 254 extends around a perimeter of the interior cavity and seals the presence transponder 256 within the interior cavity of the pouch 252. The pouch 252 is physically coupleable to an object 106 such as a piece of absorbent material. For example, the pouch 252 includes an adhesive layer 262 positioned opposite the second flexible layer 260 from the first flexible layer 258. The adhesive layer 262 may be a hot melt adhesive layer that is meltable to physically couple the pouch 252 to a piece of absorbent material but that has a melting point temperature greater than one or more sterilization temperatures at which common sterilization techniques are performed, thereby permitting the pouch 252 to remain physically coupled to the piece of absorbent material through one or multiple sterilization cycles.

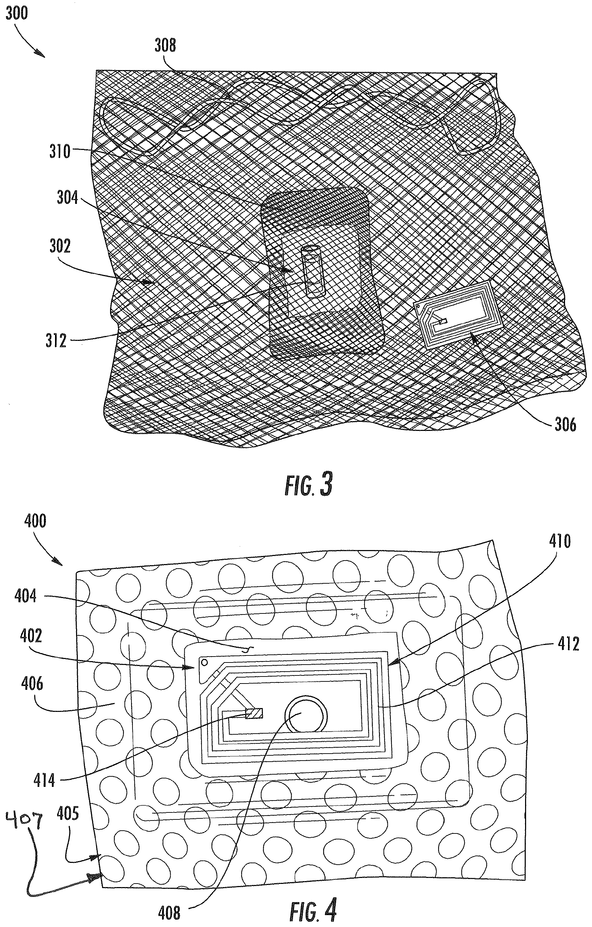

Figure 3 is a front view 300 of a piece of absorbent material 302 with a wirelessly detectable object physically coupled thereto, according to one illustrated embodiment. In particular, an RFID transponder 306 and a presence transponder 312 are physically associated with the piece of absorbent material 302.

More precisely, a pouch 304 is physically coupled to the piece of absorbent material 302. The pouch 304 includes a first flexible layer physically coupled to a second flexible layer to form an interior cavity therebetween. The flexible layers may the same as or similar to layers 208 and 210 discussed with reference to Figure 2A. The pouch 304 may include an adhesive layer that physically couples the pouch 304 to the piece of absorbent material 302. The adhesive layer may be the same as or similar to layer 212 discussed with reference to Figure 2A. In some implementations, the pouch 304 does not include the adhesive layer.

A presence transponder 312 is retained and freely movable within the interior cavity of the pouch 304. An RF weld 310 physically couples the first flexible layer to the second flexible layer. In some implementations, the RF weld 310 further physically couples the pouch 304 to the piece of absorbent material 302. In other implementations, an additional RF weld or other securing means (e.g. adhesive layer) physically couples the pouch 304 to the piece of absorbent material.

As shown in Figure 3, the RFID transponder 306 is physically coupled to the piece of absorbent material 302 separately from the pouch 304. Adhesives, stitching, clamping, fasteners, heat sealing, RF welding, or other securing means physically couple the RFID transponder 306 the piece of absorbent material 302. In some implementations, a radiopaque thread or object 308 is woven into or otherwise physically coupled to the piece of absorbent material 302, as well.

Furthermore, although Figure 3 depicts pouch 304 and RFID transponder 306 as physically coupled to and visible upon an external surface of the object piece of absorbent material 302, in some implementations, the piece of absorbent material 306 is be folded or otherwise manipulated such that the pouch 304 and RFID transponder 306 are internally carried between layers of the piece of absorbent material 302.

Figure 4 is a front view 400 of a pouch 402 that includes a presence transponder 408 freely movable within an interior cavity and an RFID transponder 410 with an antenna trace 412, according to one illustrated embodiment.

The pouch 402 includes a first flexible layer 404 physically coupled to a second flexible layer 405 to form an interior cavity therebetween. The flexible layers 404 and 405 may the same as or similar to layers 208 and 210 discussed with reference to Figure 2A. The pouch 402 includes an adhesive layer 407 physically coupled to at least the second flexible layer 405. The adhesive layer 407 may be the same as or similar to adhesive layer 212 discussed with reference to Figure 2A.

The presence transponder 408 is retained and freely movable within the interior cavity of the pouch 402. In particular, an RF weld 406 physically couples the first flexible layer 404 to the second flexible layer 405 and seals the presence transponder 408 within the interior cavity.

The RFID transponder 410 includes an antenna trace 412 electrically coupled to a chip 414. An integrated circuit that stores identification information can form all or a portion of the chip 414.

All or a portion of the RFID transponder 410 can be embedded in and/or adhered to the first flexible layer 404. For example, in some implementations, the chip 414 is adhered to the first flexible layer 404 (e.g., adhered to a surface of the first layer 404 that faces the interior cavity) while the antenna trace 412 is embedded within the first flexible layer 404. In other implementations, the antenna trace 412 is printed or traced onto the first flexible layer 404 (e.g., onto an interior surface that faces the interior cavity). In yet other implementations, all or a portion of the RFID transponder 410 is embedded in and/or adhered to the second flexible layer 405.

In some implementations, at least a portion of the first flexible layer 404 and/or the second flexible layer 405 is a material that is absorbent but remains electrically insulative, thereby contributing to an absorbency of an attached piece of absorbent material without interfering with an ability of the antenna trace 412 to transmit a signal.

As the presence transponder 408 is freely movable within the interior cavity of the pouch 402 and the RFID transponder 410 is embedded in and/or adhered to the first flexible layer 404, the presence transponder 408 is independently movable with respect to the RFID transponder 410. Furthermore, as shown in Figure 4, in some implementations, care is taken to prevent the RF weld 406 from welding over and potentially damaging the antenna trace 412. In addition, in some implementations, the pouch 402 does not include the adhesive layer 407.

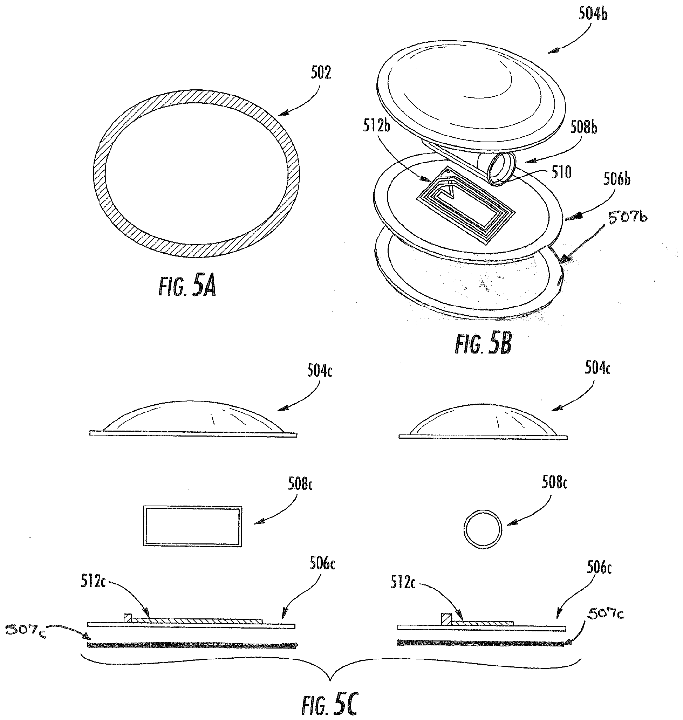

Figure 5A is a top view of a pouch 502, according to one illustrated embodiment. Figure 5B is an exploded isometric view of the pouch 502 that includes a presence transponder 508b freely movable within an interior cavity formed between a first flexible layer 504b and a substrate 506b of the pouch, according to one illustrated embodiment. An RFID transponder 512b is adhered to the substrate 506b. An encapsulant 510 encapsulates the presence transponder 508b. The substrate 506b can be a second flexible layer, a surgical object such as a piece of absorbent material, or other substrates. In particular, the first flexible layer 504b and the substrate 506b may the same as or similar to layers 208 and 210 discussed with reference to Figure 2A. In some implementations, an RF weld physically couples the first flexible layer 504b to the substrate 506b. In the illustrated embodiment, the pouch 502 further includes an adhesive layer 507b. The adhesive layer 507b may be the same as or similar to layer 212 discussed with reference to Figure 2A. However, in some implementations, the pouch 502 does not include the adhesive layer 507b

Figure 5C is first and second exploded side views of the pouch 502 that includes the presence transponder 508c freely movable within the interior cavity formed between the first flexible layer 504c and the substrate 506c of the pouch, according to one illustrated embodiment. The RFID transponder 512c is adhered to the substrate 506c of the pouch 502. For example, in some implementations, some or all of the RFID transponder 512c (e.g., a chip portion) is adhered to the substrate 506c using adhesives or other securing means. In some implementations, some or all of the RFID transponder 512c (e.g., an antenna portion) is printed onto or traced upon the substrate 506c. The illustrated pouch 502 includes the adhesive layer 507c.

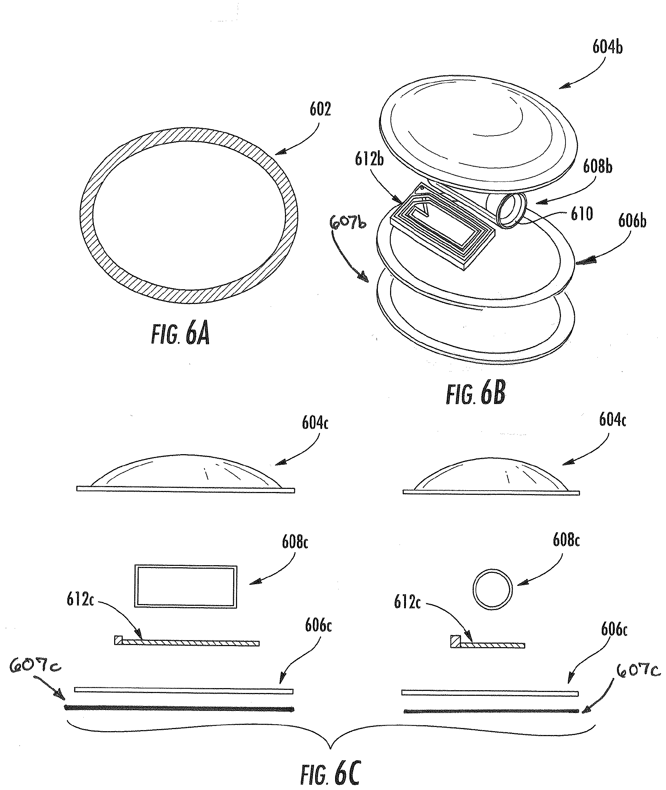

Figure 6A is a top view of a pouch 602, according to one illustrated embodiment. Figure 6B is an exploded isometric view of the pouch 602 that includes a presence transponder 608b and an RFID transponder 612b freely movable within an interior cavity formed between a first flexible layer 604b and a substrate 606b of the pouch, according to one illustrated embodiment. An encapsulant 610 encapsulates the presence transponder 608b. The substrate 606b can be a second flexible layer, a surgical object such as a piece of absorbent material, or other substrates. In particular, the first flexible layer 604b and the substrate 606b may the same as or similar to layers 208 and 210 discussed with reference to Figure 2A. In some implementations, an RF weld physically couples the first flexible layer 604b to the substrate 606b. In the illustrated embodiment, the pouch 602 further includes an adhesive layer 607b. The adhesive layer 607b may be the same as or similar to layer 212 discussed with reference to Figure 2A. However, in some implementations, the pouch 602 does not include the adhesive layer 607b.

Figure 6C is first and second exploded side views of the pouch 602 that includes the presence transponder 608c and the RFID transponder 612c freely movable within the interior cavity formed between the first flexible layer 604c and the substrate 606c of the pouch, according to one illustrated embodiment. The illustrated pouch 602 includes the adhesive layer 607c.

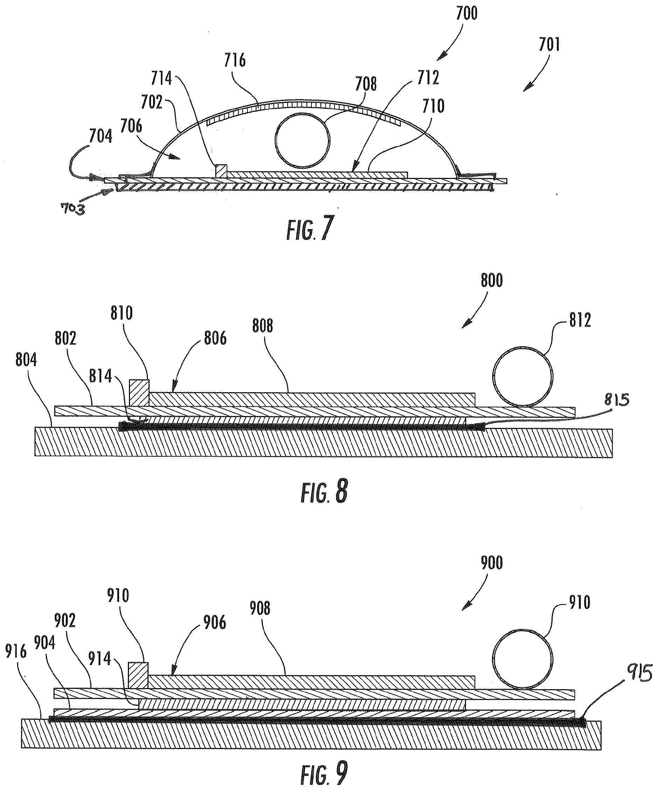

Figure 7 is a cross-sectional diagram of a wirelessly detectable object 700 that includes a directional antenna formed on or within a pouch 701, according to one illustrated embodiment. In particular, the pouch 701 includes a first flexible layer 702 physically coupled to a substrate 704 to form an interior cavity 706 therebetween. A presence transponder 708 is received and freely movable within the interior cavity 706. The substrate 704 can be a second flexible layer, a surgical object such as a piece of absorbent material, or other substrates. In particular, the first flexible layer 702 and the substrate 704 may the same as or similar to layers 208 and 210 discussed with reference to Figure 2A. In the illustrated embodiment, the wirelessly detectable object 700 further includes an adhesive layer 703. The adhesive layer 703 may be the same as or similar to layer 212 discussed with reference to Figure 2A. However, in some implementations, the wirelessly detectable object 700 does not include the adhesive layer 703.

The wirelessly detectable object further includes an RFID transponder 710 that includes at least one active antenna element 712 and an integrated circuit 714. For example, the integrated circuit 714 can actively drive or energize the active antenna element 712 of the RFID transponder 710 to transmit a signal.

According to an aspect of the present disclosure, the wirelessly detectable object 700 further includes at least one passive antenna element 716 that, together with the active antenna element 712, operates as a directional antenna. For example, the passive antenna element 716 and the active antenna element 712 may together operate as a Yagi antenna.

As shown in Figure 7, the passive antenna element 716 can be a separate structure from the active antenna element 712 of the RFID transponder 710. However, in other implementations, the passive antenna element 716 and the active antenna element 712 may be included within a single integral structure. In some implementations, two or more passive antenna elements 716 act as a reflector element and a director element, respectively.

As shown in Figure 7, the passive antenna element 716 is adhered to or traced upon an interior surface of the first flexible layer 702 that faces the interior cavity 706. However, in other implementations, the passive antenna element 716 may be at least partially embedded in the first flexible layer 702 or adhered to or traced upon an exterior surface of the first flexible layer 702. The active antenna element 712 is adhered to or traced upon an interior surface of the substrate 704 that faces the interior cavity 706. However, in other implementations, the active antenna element 712 may be at least partially embedded within the substrate 704 or adhered to or traced upon an exterior surface of the substrate 704.

In yet further implementations, the respective positions of the active antenna element 712 and the passive antenna element 716 may be opposite to those depicted in Figure 7. That is, the passive antenna element 716 may be adhered to or embedded within the substrate 704 while the active antenna element 712 is adhered to or embedded within the first flexible layer 702.

Figure 8 is a cross-sectional diagram of a wirelessly detectable object 800 that includes a directional antenna carried at least in part by a first substrate 802, according to one illustrated embodiment. The wirelessly detectable object 800 further includes an RFID transponder 806 and a presence transponder 812 physically coupled to the first substrate 802. The wirelessly detectable object 800 is physically coupled to a piece of absorbent material 804. For example, as illustrated, an adhesive layer 815 may be positioned between and respectively physically coupled to the remainder of the wirelessly detectable object 800 and the piece of absorbent material 804. However, in some implementations, the wirelessly detectable object 800 does not include the adhesive layer 815,

The first substrate 802 may be a first flexible layer. For example, the first substrate 802 may be the same as or similar to layers 208 and 210 discussed with reference to Figure 2A. The adhesive layer 815 may be the same as or similar to layer 212 discussed with reference to Figure 2A.

The RFID transponder 806 includes an active antenna element 808 and an integrated circuit 810. For example, the integrated circuit 810 may selectively actively energize or otherwise cause the active antenna element 808 to radiate to transmit a signal. The wirelessly detectable object 800 further includes at least one passive antenna element 814 that, together with the active antenna element 808, operates as a directional antenna. For example, the passive antenna element 814 and the active antenna element 808 may together operate as a Yagi antenna.

As shown in Figure 8, the passive antenna element 814 is positioned between the first substrate 802 and the piece of absorbent material 804. For example, the passive antenna element 814 can be adhered to, traced onto, or otherwise carried by one or both of the first substrate 802, the adhesive layer 815, and/or the piece of absorbent material 804. However, in other implementations, at least a portion of the passive antenna element 814 is embedded within or forms a portion of the first substrate 802 or the piece of absorbent material 804.

In yet further implementations, the respective positions of the active antenna element 808 and the passive antenna element 814 may be opposite to those depicted in Figure 8. That is, the passive antenna element 814 may be adhered to or carried by a surface of the first substrate 802 that is opposite the piece of absorbent material 804 while the active antenna element 808 is positioned between the first substrate 802 and the piece of absorbent material 804.

While Figure 8 depicts first substrate 802 as not contacting the piece of absorbent material 804 or the adhesive layer 815, in some implementations, the first substrate 802 is directly physically coupled to (e.g., by an RF weld) the piece of absorbent material 804. Further, in some implementations, the wirelessly detectable object 800 does not include the presence transponder 812.

Figure 9 is a cross-sectional diagram of a wirelessly detectable object 900 that includes a directional antenna carried at least in part by a first substrate 902, according to one illustrated embodiment. The wirelessly detectable object 900 is physically coupled to a piece of absorbent material 916. For example, as illustrated, an adhesive layer 915 may be positioned between and respectively physically coupled to the remainder of the wirelessly detectable object 900 and the piece of absorbent material 916. However, in some implementations, the wirelessly detectable object 900 does not include the adhesive layer 915.

The wirelessly detectable object 900 includes an RFID transponder 906 and a presence transponder 910 physically coupled to the first substrate 902. The wirelessly detectable object 900 further includes a second substrate 904. The first substrate 902 and/or the second substrate 904 may be flexible layers. For example, the first substrate 902 and/or the second substrate 904 may be the same as or similar to layers 208 and 210 discussed with reference to Figure 2A. The adhesive layer 915 may be the same as or similar to adhesive layer 212 discussed with reference to Figure 2A.

The RFID transponder 906 includes an active antenna element 908 and an integrated circuit 910. For example, the integrated circuit 910 may selectively actively energize or otherwise cause the active antenna element 908 to radiate to transmit a signal. The wirelessly detectable object 900 further includes at least one passive antenna element 914 that, together with the active antenna element 908, operates as a directional antenna. For example, the passive antenna element 914 and the active antenna element 908 may together operate as a Yagi antenna.

As shown in Figure 9, the passive antenna element 914 is positioned between the first substrate 902 and the second substrate 904. For example, the passive antenna element 914 can be adhered to, traced onto, or otherwise carried by one or both of the first substrate 902 and/or the second substrate 904. However, in other implementations, at least a portion of the passive antenna element 914 is embedded within or forms a portion of the first substrate 902 or the second substrate 904.

In yet further implementations, the respective positions of the active antenna element 908 and the passive antenna element 914 may be opposite to those depicted in Figure 9. That is, the passive antenna element 914 may be adhered to or carried by a surface of the first substrate 902 that is opposite the second substrate 904 while the active antenna element 908 is positioned between the first substrate 902 and the second substrate 904. Further, in some implementations, one or more RF welds or other securing means (e.g., adhesive layer 915) physically couple one or both of the first and second substrates 902 and 904 to the piece of absorbent material 916.

Furthermore, while Figure 9 depicts first substrate 802 as not directly contacting the second substrate 904, in some implementations, the first substrate 902 is directly physically coupled to (e.g., by an RF weld) the second substrate 904. Likewise, an RF weld may physically couple the second substrate 904 to the piece of absorbent material. Further, in some implementations, the wirelessly detectable object 900 does not include the presence transponder 910.

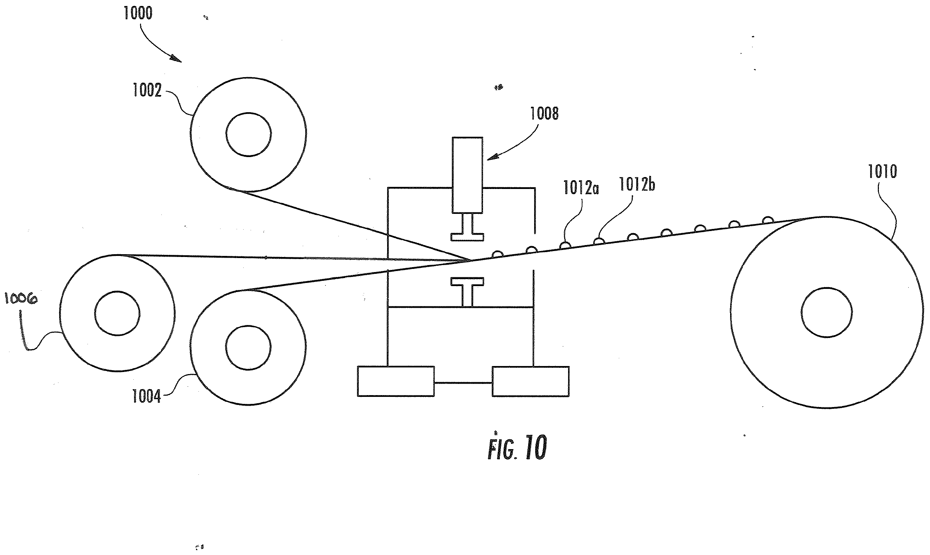

Figure 10 is a schematic diagram 1000 of an exemplary method for manufacturing wirelessly detectable objects using RF welding, according to one illustrated embodiment. In particular, the method may include providing a first flexible layer 1002; a second flexible layer 1006; and an adhesive layer 1004. For example, either or both of the first flexible layer 1002 and the second flexible layer 1006 may be the same as or similar to layers 208 and 210 discussed with reference to Figure 2A. The adhesive layer 1004 may be the same as or similar to adhesive layer 212 of Figure 2A. In some implementations, as shown in Figure 10, the first and/or second flexible layers 1002 and 1006 may be provided as rolls or sheets of flexible layers. Further, in some implementations, a roll of absorbent material (not shown) may also be provided.

The exemplary method may further include RF welding the first flexible layer 1002 to the second flexible layer 1006 to form a plurality of pouches (e.g., pouches 1012a and 1012b). The adhesive layer 1004 may be physically coupled (e.g., by RF welding or other techniques) to at least the second flexible layer 1006 opposite the first flexible layer 1002.

Each of the plurality of pouches can be formed by a set of RF welds. For example, an RF welding machine 1008 can be used to create a plurality of RF welds that physically couple the first flexible layer 1002 to the second flexible layer 1006 and create the plurality of pouches 1012a and 1012b. Each set of RF welds can take the form of a hollowed rectangle, circle, oval, or other shape to form an interior cavity within a perimeter of the hollowed area. One or more transponders can be sealed within the interior cavity of each pouch 1012.

Thus, through automatic or manual operation of the RF welding machine 1008 to generate the plurality of RF welds, the first and second flexible layers 1002 and 1006 are transformed into a sheet or roll of pouches 1010, with each pouch 1012 retaining one or more transponders. As such, rather than being discretely made from the assembly of individual components, the pouches 1012 may come as a roll of pouches 1010 each containing one or more respective transponders. Having the pouches 1012 come in a roll 1010 enhances the efficiency in the manufacturing process, as all that remains to be done is cutting or separating the pouches 1012 from the roll 1010 and attaching each of the pouches 1012 to a respective surgical object (e.g., via adhesive layer 1004).

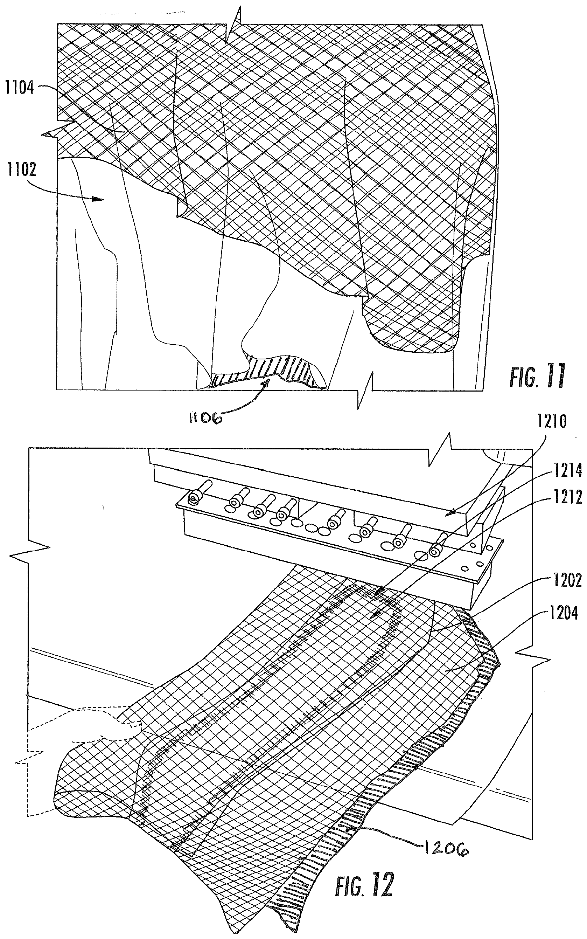

Figure 11 shows flexible layers usable to manufacture a plurality of pouches, according to one illustrated embodiment. In particular, Figure 11 shows a first flexible layer 1104 of nylon; a second flexible layer 1102 of thermoplastic polyurethane; and an adhesive layer 1106. The above noted materials are provided as examples only. In particular, the flexible layers 1104 and 1102 may be the same as or similar to layers 208 and 210 discussed with reference to Figure 2A.

Figure 12 shows manufacture of a plurality of pouches using an RF welding technique, according to one illustrated embodiment. In particular, Figure 12 shows the first flexible layer 1204 of nylon; the second flexible layer 1202 of thermoplastic polyurethane; and the adhesive layer 1206. An RF welding machine 1210 is used to generate a plurality of RF welds to physically couple layer 1102 to layer 1104 and/or adhesive layer 1206 and form a plurality of pouches. As an example, an RF weld 1214 forms at least a portion of a perimeter of an interior cavity of an unfinished pouch 1212. One or more transponders (not shown) may be positioned between layers 1102 and 1104 and then sealed within the pouch 1212 by an additional RF weld.

As one example method of manufacture, the pouches may be made by RF welding the first layer 1204 to the second layer 1202 where a series of cavities for receiving one or more corresponding transponders are made by providing bulges in the first layer 1204 and/or the second layer 1202. The bulges may be formed by bunching or stretching the material of the first layer 1204 and/or the second layer 1202.

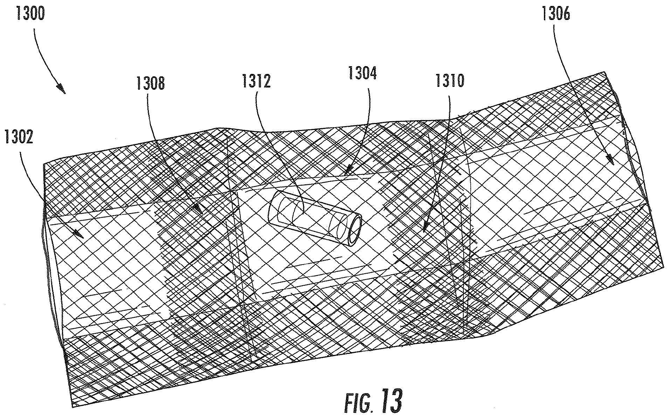

Figure 13 is a front view 1300 of a plurality of pouches 1302, 1304, and 1306 manufactured using the RF welding technique illustrated in Figures 10 and 12, according to one illustrated embodiment. In particular, a plurality of RF welds form each of pouches 1302, 1304, and 1306. For example, RF welds 1308 and 1310 form at least a portion of a perimeter of an interior cavity of pouch 1304. A presence transponder 1312 is received and freely movable within the interior cavity of pouch 1304. Pouches 1302 and 1306 are bisected for the purposes of illustration. The pouches 1302, 1304, and 1306 may be physically separated (e.g., cut apart) and then respectively physically coupled to surgical objects to act as wirelessly detectable objects (e.g., via use of an adhesive layer).

The above description of illustrated embodiments, including what is described in the Abstract, is not intended to be exhaustive or to limit the various embodiments to the precise forms disclosed. Although specific embodiments of and examples are described herein for illustrative purposes, various equivalent modifications can be made without departing from the scope of the invention defined by the appended claims, as will be recognized by those skilled in the relevant art.

The teachings provided herein can be applied to other absorbent materials, other types of transponders, and other interrogation and detection systems. For instance, the transponder device may be used to mark objects anytime detection of the presence of marked objects is desirable in a confined area, not just during surgery. For example, it may be used to make sure marked objects are not left inside a machine (e.g., vehicle, copy machine) after maintenance is performed. In at least some embodiments, the transponder housing may be utilized to mark objects to determine the removal of a marked object from a confined area, such as a cover-all garment from a clean room of a semiconductor fabrication plant. In such an embodiment, an interrogation device, for example, may be placed proximate to a door of the confined area.

In addition, a transponder pouch may be manufactured and distributed for tagging objects without a transponder currently attached or received therein. Advantageously, the pouch can then be used to place a transponder compatible with a particular detection and interrogation system at a subsequent time, including by the end-user.

These and other changes can be made in light of the above-detailed description. In general, in the following claims, the terms used should not be construed to limit the invention to the specific embodiments disclosed in the specification and the claims, but should be construed to include all possible embodiments along with the full scope of equivalents to which such claims are entitled. Accordingly, the invention is not limited by the disclosure, but is defined by the scope of the claims appended hereto.

## Claims

1. A wirelessly detectable object (118; 700) to use in medical procedures, comprising:
at least a first transponder (206; 708, 710) that wirelessly receives a first interrogation signal and wirelessly returns a first response signal; and
a surgical object;
comprising a pouch (202; 701) comprising at least a first flexible layer (208; 702) that forms an interior cavity (706) and an adhesive layer (212; 703) physically coupled to at least the first flexible layer (208; 702),
wherein the pouch (202; 701) is physically coupled to at least a portion of the surgical object and
**characterised in that**,
the adhesive layer (212; 703) is about a perimeter of the interior cavity (706), and
wherein the adhesive layer (212; 703) retains structural and adhesive integrity at least at temperatures equal to one of 121 degrees Centigrade, 130 degrees Centigrade, 136 degrees Centigrade and 150 degrees Centigrade.

2. The wirelessly detectable object (118; 700) of claim 1, wherein the surgical object comprises a piece of absorbent material.

3. The wirelessly detectable object (118; 700) of claim 1 or 2, wherein the adhesive layer (212; 703) physically couples the pouch (202; 701) to at least the portion of the surgical object.

4. The wirelessly detectable object (118) of one of claims 1 to 3, wherein the interior cavity is formed between the first flexible layer (208) and the adhesive layer (212).

5. The wirelessly detectable object (118; 700) of one of claims 1 to 4, further comprising: a second flexible layer (210; 704) physically coupled to the first flexible layer (208; 702), the interior cavity (706) formed between the first flexible layer (208; 702) and the second flexible layer (210; 704).

6. The wirelessly detectable object (118; 700) of claim 5, wherein the second flexible layer (210; 704) is physically coupled to the adhesive layer (212; 703).

7. The wirelessly detectable object (118) of claim 5, wherein the adhesive layer (212) is physically coupled to at least a portion of a first surface of the first flexible layer (208) and the second flexible layer (210) is physically coupled to at least a portion of a second surface of the first flexible layer (208) that is opposite the first surface.

8. The wirelessly detectable object of claim 5, wherein the adhesive layer is physically coupled to at least a first surface of the first flexible layer about the perimeter of the interior cavity and the second flexible layer is physically coupled to at least a second surface of the first flexible layer about the perimeter of the interior cavity, the second surface of the first flexible layer opposite the first surface of the first flexible layer.

9. The wirelessly detectable object of claim 5, wherein the adhesive layer is continuously physically coupled to at least a first surface of the first flexible layer and the second flexible layer is physically coupled to at least a second surface of the first flexible layer about the perimeter of the interior cavity, the second surface of the first flexible layer opposite the first surface of the first flexible layer.

10. The wirelessly detectable object (118; 700) of claim 1 wherein the adhesive layer (212; 703) comprises a hot melt adhesive layer, in particular a high temperature hot melt adhesive layer.

11. The wirelessly detectable object (118; 700) of claim 10, wherein the hot melt adhesive layer has a melting point temperature greater than a sterilization temperature associated with one or more sterilization procedures, in particular a melting point temperature greater than a steam temperature at which a volume of steam is maintained during one or more sterilization procedures.

12. The wirelessly detectable object (118; 700) of claim 10 or 11, wherein the hot melt adhesive layer has a melting point temperature greater than 136 degrees Centigrade, preferably of about 150 degrees Centigrade or higher.

13. The wirelessly detectable object (118; 700) of one of claims 1 to 12, wherein the adhesive layer (212; 703) is biocompatible.

14. The wirelessly detectable object (118; 700) of one of claims 1 to 13, wherein the adhesive layer (212; 703) comprises at least one of a meltable plastic layer, a thermoplastic layer, an adhesive web film, a thermal lamination film, a heat-activated adhesive layer, a pressure-activated adhesive layer, a water-activated adhesive layer and a thermosetting plastic layer that has an initial cure temperature at which the thermosetting plastic layer cures, the thermosetting plastic layer which retains structural and adhesive integrity at least at temperatures equal to 121 degrees Centigrade subsequent to curing.

15. The wirelessly detectable object (118; 700) of one of claims 1 to 14, wherein the adhesive layer (212; 703) comprises at least one of thermoplastic polyurethane, silicone, polyamide, polyethersulfone, polyethylene, polypropylene, and ethylene vinyl acetate.

16. The wirelessly detectable object (118; 700) of one of claims 1 to 15, wherein the first transponder (206; 708) is received and freely movable in the interior cavity (706).

17. The wirelessly detectable object (118; 700) of one of claims 1 to 16, wherein the first transponder comprises a presence transponder (206; 708) that wirelessly receives the first interrogation signal and wirelessly returns the first response signal that does not contain identification information.

18. The wirelessly detectable object (700) of one of claims 1 to 16, wherein the first transponder comprises a radio frequency identification (RFID) transponder (710) that wirelessly receives the first interrogation signal and wirelessly returns the first response signal that contains identification information associated with the wirelessly detectable object (700).

19. The wirelessly detectable object (700) of claim 17, further comprising: a radio frequency identification (RFID) transponder (710) that wirelessly receives a second interrogation signal and wirelessly returns a second response signal that contains identification information associated with the wirelessly detectable object (700), the presence transponder (708) not directly physically attached to the RFID transponder (710).

20. The wirelessly detectable object (700) of claim 19, wherein the RFID transponder (710) is received within the interior cavity (706), preferably freely movable within the interior cavity.

21. The wirelessly detectable object of claim 19, wherein the RFID transponder forms at least a portion of the first flexible layer, is embedded within the first flexible layer, or is adhered to the first flexible layer by the adhesive layer.

22. The wirelessly detectable object (118; 700) of claim 1 or claim 5, wherein the pouch (202; 701) further comprises a radio frequency (RF) weld (204) that extends around a perimeter of the interior cavity (706), physically couples the first flexible layer (208; 702) to the adhesive layer (212; 703) or to the second flexible layer (210; 704), and seals the presence transponder (206; 708) within the interior cavity (706).

23. The wirelessly detectable object (118; 700) of claim 5, wherein one or both of the first flexible layer (208; 702) and second flexible layer (210; 704) are a fabric laminate, in particular a fabric laminate comprising thermoplastic polyurethane and nylon fabric or polyvinyl chloride (PVC) impregnated fabric.

## Patentansprüche

1. Drahtlos detektierbarer Gegenstand (118; 700) zur Verwendung bei medizinischen Verfahren, umfassend:
mindestens einen ersten Transponder (206; 708, 710), der ein erstes Abfragesignal drahtlos empfängt und ein erstes Antwortsignal drahtlos zurücksendet, und
einen chirurgischen Gegenstand,
umfassend:
eine Tasche (202, 701), die mindestens eine erste flexible Schicht (208; 702), welche einen inneren Hohlraum (706) bildet, und eine Klebstoffschicht (212; 703) umfasst, die zumindest an die erste flexible Schicht (208; 702) physisch gekoppelt ist,
wobei die Tasche (202; 701) zumindest an einen Teil des chirurgischen Gegenstandes physisch gekoppelt ist,
**dadurch gekennzeichnet, dass**
die Klebstoffschicht (212; 703) ungefähr einen Umfang des inneren Hohlraums (706) aufweist,
wobei die Klebstoffschicht (212; 703) zumindest bei Temperaturen, die gleich einer von 121 Grad Celsius, 130 Grad Celsius, 136 Grad Celsius und 150 Grad Celsius sind, strukturelle und adhäsive Integrität behält.

2. Drahtlos detektierbarer Gegenstand (118; 700) nach Anspruch 1, wobei der chirurgische Gegenstand ein Absorptionsmaterialstück umfasst.

3. Drahtlos detektierbarer Gegenstand (118; 700) nach Anspruch 1 oder 2, wobei die Klebstoffschicht (212; 703) die Tasche (202; 701) zumindest an den Teil des chirurgischen Gegenstands physisch koppelt.

4. Drahtlos detektierbarer Gegenstand (118) nach einem der Ansprüche 1 bis 3, wobei der innere Hohlraum zwischen der ersten flexiblen Schicht (208) und der Klebstoffschicht (212) gebildet ist.

5. Drahtlos detektierbarer Gegenstand (118; 700) nach einem der Ansprüche 1 bis 4, ferner umfassend: eine zweite flexible Schicht (210; 704), die an die erste flexible Schicht (208; 702) physisch gekoppelt ist, wobei der innere Hohlraum (706) zwischen der ersten flexiblen Schicht (208; 702) und der zweiten flexiblen Schicht (210; 704) gebildet ist.

6. Drahtlos detektierbarer Gegenstand (118; 700) nach Anspruch 5, wobei die zweite flexible Schicht (210; 704) an die Klebstoffschicht (212; 703) physisch gekoppelt ist.

7. Drahtlos detektierbarer Gegenstand (118) nach Anspruch 5, wobei die Klebstoffschicht (212) zumindest an einen Teil einer ersten Oberfläche der ersten flexiblen Schicht (208) physisch gekoppelt ist und die zweite flexible Schicht (210) zumindest an einen Teil einer zweiten Oberfläche der ersten flexiblen Schicht (208) physisch gekoppelt ist, die der ersten Oberfläche gegenüberliegt.

8. Drahtlos detektierbarer Gegenstand nach Anspruch 5, wobei die Klebstoffschicht zumindest an eine erste Oberfläche der ersten flexiblen Schicht um den Umfang des inneren Hohlraums physisch gekoppelt ist und die zweite flexible Schicht zumindest an eine zweite Oberfläche der ersten flexiblen Schicht um den Umfang des inneren Hohlraums physisch gekoppelt ist, wobei die zweite Oberfläche der ersten flexiblen Schicht gegenüber der ersten Oberfläche der ersten flexiblen Schicht liegt.

9. Drahtlos detektierbarer Gegenstand nach Anspruch 5, wobei die Klebstoffschicht zumindest an eine erste Oberfläche der ersten flexiblen Schicht durchgehend physisch gekoppelt ist und die zweite flexible Schicht zumindest an eine zweite Oberfläche der ersten flexiblen Schicht um den Umfang des inneren Hohlraums physisch gekoppelt ist, wobei die zweite Oberfläche der ersten flexiblen Schicht gegenüber der ersten Oberfläche der ersten flexiblen Schicht liegt.

10. Drahtlos detektierbarer Gegenstand (118; 700) nach Anspruch 1, wobei die Klebstoffschicht (212; 703) eine Heißkleberschicht, insbesondere eine Hochtemperatur-Heißkleberschicht umfasst.

11. Drahtlos detektierbarer Gegenstand (118; 700) nach Anspruch 10, wobei die Heißkleberschicht eine Schmelzpunkttemperatur aufweist, die größer als eine Sterilisationstemperatur ist, die mit einem oder mehreren Sterilisationsverfahren verbunden ist, insbesondere eine Schmelzpunkttemperatur, die größer als eine Dampftemperatur ist, auf der ein Dampfvolumen während eines oder mehrerer Sterilisationsverfahren gehalten wird.

12. Drahtlos detektierbarer Gegenstand (118; 700) nach Anspruch 10 oder 11, wobei die Heißkleberschicht eine Schmelzpunkttemperatur aufweist, die größer als 136 Grad Celsius, vorzugsweise ungefähr 150 Grad Celsius oder höher ist.

13. Drahtlos detektierbarer Gegenstand (118; 700) nach einem der Ansprüche 1 bis 12, wobei die Klebstoffschicht (212; 703) biokompatibel ist.

14. Drahtlos detektierbarer Gegenstand (118; 700) nach einem der Ansprüche 1 bis 13, wobei die Klebstoffschicht (212; 703) mindestens eine von einer schmelzbaren Kunststoffschicht, einer thermoplastischen Schicht, einer klebenden Gewebefolie, einer thermischen Laminierfolie, einer wärmeaktivierten Klebstoffschicht, einer druckaktivierten Klebstoffschicht, einer wasseraktivierten Klebstoffschicht und einer wärmehärtenden Kunststoffschicht, die eine anfängliche Aushärtungstemperatur aufweist, bei der die wärmehärtende Kunststoffschicht aushärtet, umfasst, wobei die wärmehärtende Kunststoffschicht im Anschluss an das Aushärten mindestens bei Temperaturen gleich 121 Grad Celsius Struktur- und Klebstoffintegrität beibehält.

15. Drahtlos detektierbarer Gegenstand (118; 700) nach einem der Ansprüche 1 bis 14, wobei die Klebstoffschicht (212; 703) mindestens eines von thermoplastischem Polyurethan, Silikon, Polyamid, Polyethersulfon, Polyethylen, Polypropylen und Ethylenvinylacetat umfasst.

16. Drahtlos detektierbarer Gegenstand (118; 700) nach einem der Ansprüche 1 bis 15, wobei der erste Transponder (206; 708) in dem inneren Hohlraum (706) aufgenommen und frei beweglich ist.

17. Drahtlos detektierbarer Gegenstand (118; 700) nach einem der Ansprüche 1 bis 16, wobei der erste Transponder einen Anwesenheitstransponder (206; 708) umfasst, der das erste Abfragesignal drahtlos empfängt und das erste Antwortsignal, das keine Identifizierungsinformationen enthält, drahtlos zurücksendet.

18. Drahtlos detektierbarer Gegenstand (700) nach einem der Ansprüche 1 bis 16, wobei der erste Transponder einen Radiofrequenzidentifikations-(RFID)-Transponder (710) umfasst, der das erste Abfragesignal drahtlos empfängt und das erste Antwortsignal, das mit dem drahtlos detektierbaren Gegenstand (700) assoziierte Identifikationsinformationen enthält, drahtlos zurücksendet.

19. Drahtlos detektierbarer Gegenstand (700) nach Anspruch 17, ferner umfassend: einen Radiofrequenzidentifikations-(RFID)-Transponder (710), der drahtlos ein zweites Abfragesignal empfängt und drahtlos ein zweites Antwortsignal, das mit dem drahtlos detektierbaren Gegenstand (700) assoziierte Identifikationsinformationen enthält, zurücksendet, wobei der Anwesenheitstransponder (708) nicht direkt physisch mit dem RFID-Transponder (710) verbunden ist.

20. Drahtlos detektierbarer Gegenstand (700) nach Anspruch 19, wobei der RFID-Transponder (710) innerhalb des inneren Hohlraums (706), vorzugsweise innerhalb des inneren Hohlraums frei beweglich, aufgenommen ist.

21. Drahtlos detektierbarer Gegenstand nach Anspruch 19, wobei der RFID-Transponder zumindest einen Teil der ersten flexiblen Schicht bildet, in die erste flexible Schicht eingebettet ist oder mittels der Klebstoffschicht an die erste flexible Schicht angeklebt ist.

22. Drahtlos detektierbarer Gegenstand (118; 700) nach Anspruch 1 oder Anspruch 5, wobei die Tasche (202; 701) ferner eine Radiofrequenz-(RF)-Schweißnaht (204) aufweist, die sich um einen Umfang des inneren Hohlraums (706) erstreckt, die erste flexible Schicht (208; 702) an die Klebstoffschicht (212; 703) oder die zweite flexible Schicht (210; 704) physisch koppelt und den Anwesenheitstransponder (206; 708) in dem inneren Hohlraum (706) abdichtet.

23. Drahtlos detektierbarer Gegenstand (118; 700) nach Anspruch 5, wobei eine oder beide der ersten flexiblen Schicht (208; 702) und der zweiten flexiblen Schicht (210; 704) ein Gewebelaminat ist/sind, insbesondere ein Gewebelaminat, das thermoplastisches Polyurethan und Nylongewebe oder mit Polyvinylchlorid (PVC) imprägniertes Gewebe umfasst.

## Revendications

1. Objet détectable sans fil (118; 700) pour l'utilisation dans des procédés médicaux, comprenant:
au moins un premier transpondeur (206; 708, 710) qui reçoit sans fil un premier signal d'interrogation et renvoie sans fil un premier signal de réponse, et
un objet chirurgical,
comprenant:
une poche (202; 701) comprenant au moins une première couche souple (208; 702) qui forme une cavité intérieure (706), et une couche adhésive (212; 703) couplée physiquement à au moins la première couche souple (208; 702),
dans lequel ladite poche (202; 701) est couplée physiquement à au moins une partie de l'objet chirurgical,
**caractérisé en ce que**
ladite couche adhésive (212; 703) présente à peu près un périmètre de la cavité intérieure (706), et
dans lequel la couche adhésive (212; 703) conserve de l'intégrité structurale et adhésive au moins à des températures égales à l'une de 121 degrés Celsius, de 130 degrés Celsius, de 136 degrés Celsius et de 150 degrés Celsius.

2. Objet détectable sans fil (118; 700) selon la revendication 1, dans lequel l'objet chirurgical comprend une pièce de matière absorbante.

3. Objet détectable sans fil (118; 700) selon la revendication 1 ou 2, dans lequel la couche adhésive (212; 703) couple physiquement la poche (202; 701) à au moins la partie de l'objet chirurgical.

4. Objet détectable sans fil (118) selon l'une quelconque des revendications 1 à 3, dans lequel la cavité intérieure est formée entre la première couche souple (208) et la couche adhésive (212).

5. Objet détectable sans fil (118; 700) selon l'une quelconque des revendications 1 à 4, comprenant en outre: une deuxième couche souple (210; 704) couplée physiquement à la première couche souple (208; 702), la cavité intérieure (706) étant formée entre la première couche souple (208; 702) et la deuxième couche souple (210; 704).

6. Objet détectable sans fil (118; 700) selon la revendication 5, dans lequel la deuxième couche souple (210; 704) est couplée physiquement à la couche adhésive (212; 703).

7. Objet détectable sans fil (118) selon la revendication 5, dans lequel la couche adhésive (212) est couplée physiquement à au moins une partie d'une première surface de la première couche souple (208) et la deuxième couche souple (210) est couplée physiquement à au moins une partie d'une deuxième surface de la première couche souple (208), qui est opposée à la première surface.

8. Objet détectable sans fil selon la revendication 5, dans lequel la couche adhésive est couplée physiquement à au moins une première surface de la première couche souple autour du périmètre de la cavité intérieure, et la deuxième couche souple est couplée physiquement à au moins une deuxième surface de la première couche souple autour du périmètre de la cavité intérieure, la deuxième surface de la première couche souple étant opposée à la première surface de la première couche souple.

9. Objet détectable sans fil selon la revendication 5, dans lequel la couche adhésive est couplée physiquement en continu à au moins une première surface de la première couche souple et la deuxième couche souple est couplée physiquement à au moins une deuxième surface de la première couche souple autour du périmètre de la cavité intérieure, la deuxième surface de la première couche souple étant opposée à la première surface de la première couche souple.

10. Objet détectable sans fil (118; 700) selon la revendication 1, dans lequel la couche adhésive (212; 703) comprend une couche d'adhésif thermofusible, en particulier une couche d'adhésif thermofusible à haute température.

11. Objet détectable sans fil (118; 700) selon la revendication 10, dans lequel la couche d'adhésif thermofusible présente une température de point de fusion supérieure à une température de stérilisation associée à une ou plusieurs procédures de stérilisation, en particulier une température de point de fusion supérieure à une température de vapeur à laquelle un volume de vapeur est maintenu pendant une ou plusieurs procédures de stérilisation.

12. Objet détectable sans fil (118; 700) selon la revendication 10 ou 11, dans lequel la couche d'adhésif thermofusible présente une température de point de fusion supérieure à 136 degrés Celsius, de préférence d'à peu près 150 degrés Celsius ou plus.

13. Objet détectable sans fil (118; 700) selon l'une quelconque des revendications 1 à 12, dans lequel la couche adhésive (212; 703) est biocompatible.

14. Objet détectable sans fil (118; 700) selon l'une quelconque des revendications 1 à 13, dans lequel la couche adhésive (212; 703) comprend au moins l'un(e) parmi une couche en matière plastique fusible, une couche thermoplastique, un film de tissu adhésif, un film de stratification thermique, une couche adhésive activée par la chaleur, une couche adhésive activée par la pression, une couche adhésive activée par l'eau et une couche en matière plastique thermodurcissable ayant une température de durcissement initiale à laquelle la couche en matière plastique thermodurcissable durcit, la couche en matière plastique thermodurcissable conservant de l'intégrité structurelle et adhésive suite au durcissement, au moins à des températures égales à 121 degrés Celsius.

15. Objet détectable sans fil (118; 700) selon l'une quelconque des revendications 1 à 14, dans lequel la couche adhésive (212; 703) comprend au moins l'un parmi le polyuréthane thermoplastique, le silicone, le polyamide, le polyéthersulfone, le polyéthylène, le polypropylène et l'éthylène-acétate de vinyle.

16. Objet détectable sans fil (118; 700) selon l'une quelconque des revendications 1 à 15, dans lequel le premier transpondeur (206; 708) est reçu et librement déplaçable à l'intérieur de la cavité intérieure (706).

17. Objet détectable sans fil (118; 700) selon l'une quelconque des revendications 1 à 16, dans lequel le premier transpondeur comprend un transpondeur de présence (206; 708) qui reçoit sans fil le premier signal d'interrogation et renvoie sans fil le premier signal de réponse qui ne contient pas d'information d'identification.

18. Objet détectable sans fil (700) selon l'une quelconque des revendications 1 à 16, dans lequel le premier transpondeur comprend un transpondeur d'identification radiofréquence (RFID) (710) qui reçoit sans fil le premier signal d'interrogation et renvoie sans fil le premier signal de réponse contenant des informations d'identification associées à l'objet détectable sans fil (700).

19. Objet détectable sans fil (700) selon la revendication 17, comprenant en outre: un transpondeur d'identification radiofréquence (RFID) (710) qui reçoit sans fil un deuxième signal d'interrogation et renvoie sans fil un deuxième signal de réponse contenant des informations d'identification associées à l'objet détectable sans fil (700), ledit transpondeur de présence (708) n'étant pas relié directement physiquement au transpondeur RFID (710).

20. Objet détectable sans fil (700) selon la revendication 19, dans lequel ledit transpondeur RFID (710) est reçu à l'intérieur de la cavité intérieure (706), de préférence est déplaçable librement à l'intérieur de la cavité intérieure.

21. Objet détectable sans fil selon la revendication 19, dans lequel le transpondeur RFID forme au moins une partie de la première couche souple, est encastré dans la première couche souple ou est collé à la première couche souple par la couche adhésive.

22. Objet détectable sans fil (118; 700) selon la revendication 1 ou la revendication 5, dans lequel la poche (202; 701) comprend en outre une soudure radiofréquence (RF) (204) qui s'étend autour d'un périmètre de la cavité intérieure (706), couple physiquement la première couche souple (208; 702) à la couche adhésive (212; 703) ou à la deuxième couche souple (210; 704) et rend étanche le transpondeur de présence (206; 708) dans la cavité intérieure (706).

23. Objet détectable sans fil (118; 700) selon la revendication 5, dans lequel une ou les deux des première (208; 702) et deuxième (210; 704) couches souples est/sont un stratifié de tissu, en particulier un stratifié de tissu comprenant du polyuréthane thermoplastique et du tissu de nylon ou un tissu imprégné de polychlorure de vinyle (PVC).
